# EUROPÄISCHE PATENTSCHRIFT

(11) **EP 2 532 638 B1**
(45) Veröffentlichungstag und Bekanntmachung des Hinweises auf die Patenterteilung: **19.08.2015**
(21) Anmeldenummer: 11004626.5
(22) Anmeldetag: 07.06.2011
(51) Int. Cl.: C07C 5/22, C07C 13/18

(54) **Verfahren zur Isomerisierung**
Isomerisation method
Procédé d'isomérisation

(43) Veröffentlichungstag der Anmeldung: 12.12.2012
(73) Patentinhaber: Merck Patent GmbH, 64293 Darmstadt (DE)
(72) Erfinder: Wächtler, Andreas, Dr., 64295 Darmstadt (DE); Schäfer, Ralf, 63225 Langen (DE); Ambrosius, Klaus, Dr., 64807 Dieburg (DE)

(56) Entgegenhaltungen:
- DE-A1-102005 034 067
- DE-A1-102009 058 573
- DATABASE WPI Week 199725 Thomson Scientific, London, GB; AN 1997-276717 XP000002659224, & JP 9 100286 A (SHINETSU CHEM IND CO LTD) 15. April 1997 (1997-04-15)
- DATABASE WPI Week 200463 Thomson Scientific, London, GB; AN 2004-646561 XP000002659225, & JP 2004 256490 A (NAGASE CIBA KK) 16. September 2004 (2004-09-16)
- DATABASE WPI Week 199336 Thomson Scientific, London, GB; AN 1993-285371 XP000002659304, & JP 5 201881 A (CHISSO CORP) 10. August 1993 (1993-08-10)

## Beschreibung

Die vorliegende Erfindung betrifft ein Verfahren zur Herstellung von all-trans-4,4'-disubstituierten Bicyclohexanderivaten und deren Analoga, das einen Isomerisierungsschritt umfasst, der einen oder mehrere ciskonfigurierte Cyclohenxanringe unter Verwendung einer Lewis-Säure oder einer Lewis-sauren ionischen Flüssigkeit in die trans-Konfiguration überführt.

Funktionale Chemikalien werden nicht nur durch ihre vorhandenen funktionellen Gruppen, sondern auch maßgeblich durch ihre Stereochemie, also ihrem räumlichen Aufbau, beeinflusst. Ein nicht unwesentlicher Teilbereich der Stereochemie ist einfachen nicht-aromatischen Ringen und der relativen Lage von Substituenten an diesen Ringen gewidmet.

Ein Grundproblem für die Stereochemie stellt die cis-trans-Isomerie von Substituenten an Cyclohexanen dar. Im Folgenden wird speziell auf die Isomerie von 1,4-substituierten Cyclohexanderivaten eingegangen. Obwohl für die Einstellung der Stereochemie von funktionalisierten Cyclohexanen (z.B. Cyclohexancarbonsäuren, Cyclohexylcarbaldehyden, Cyclohexanolen oder auch Phenylcyclohexanen) zahlreiche Methoden existieren, ist die Stereochemie von rein aliphatischen nichtfunktionalisierten Cyclohexanen relativ schwierig zu steuern. Dass hierfür noch keine allgemein anwendbare Lösung existiert, ist insofern erstaunlich, als dass Verbindungen dieses Typs für technische Anwendungen schon seit langem bekannt sind (vgl. JP 59070624 A, 1984) und diese wegen des cis/trans-Problems aufwendig über die funktionalisierten Cyclohexanverbindungen hergestellt werden (vgl. CN 1962580 A).

Eine bekannte Reaktion ist die cis-trans-Isomerisierung von (4-Alkyl-cyclohexyl)-benzolen. Dabei lässt sich der phenylsubstituierte Cyclohexanring durch geeignete Maßnahmen dahingehend isomerisieren, dass aus einem cis-trans-Gemisch überwiegend das 1,4-trans-Isomer erhalten wird (JP 2004-256490 A). Im Gegensatz zum Gegenstand der vorliegenden Erfindung kann hierbei die Isomerisierung in Benzylstellung erfolgen. Die benzylische Position ist vergleichsweise leicht zur Isomerisierung zu bewegen. Daher verwenden z.B. herkömmliche Syntheserouten zur Herstellung von trans-Bicyclohexanen grundsätzlich Cyclohexylbenzol-Zwischenstufen, so dass wenigstens bereits einer der Cyclohexanringe in die trans-Konfiguration gebracht werden kann. An 4,4'-dialkylierten Bicyclohexanen wurde bisher keine cis/trans-Isomerisierungen beschrieben.

Für Isomerisierungsverfahren bei Phenylcyclohexanen werden bislang starke Basen wie Kalium-tert-butylat oder bei funktionalen Cyclohexanderivaten beispielsweise Fluoridionen (DE102005034067 A1) eingesetzt. Diese Isomerisierungsverfahren versagen jedoch bei rein aliphatischen 4,4'-Dialkylbicyclohexanen.

Die Druckschriften JP 09-100286 A und JP 05-201881 A offenbaren die cis-trans-Isomerisierung von 1,4-substituierten Cyclohexanen unter dem Einfluss von Lewis-Säuren.

Es wurde nun ein allgemein anwendbares Verfahren zur Herstellung von 1,4-disubstituierten Cyclohexanverbindungen mit all-trans Konfiguration der allgemeinen Formel I gefunden,

R¹-[A¹-(CH₂)ₙ-]ₘ-A²-R² I

worin
- R¹ und R²: unabhängig voneinander einen unsubstituierten, geradkettigen Alkylrest mit bis zu 9 C-Atomen oder -CH(CH₃)₂,
- m: 1 oder 2,
- n: 0, 1, 2, 3 oder 4, bevorzugt 0, 2 oder 4 und
- A¹, A²: *trans*-1,4-Cyclohexyl,
bedeuten, das die Umsetzung von Verbindungen der Formel I entsprechenden 1,4-cis-Cyclohexanverbindungen in Gegenwart
a) einer Lewis-Säure oder
b) eines Lewis-sauren Anions,
und in Gegenwart einer zusätzlichen Komponente, die zusammen mit der Lewis-Säure bzw. mit dem Lewis-sauren Anion ein Carbokation bilden kann, umfasst, dadurch gekennzeichnet, dass es in Gegenwart eines sekundären oder tertiären Alkylchlorids oder Alkylbromids, eines Acyl- oder Aroylhalogenids oder eines primären Halogenids mit Neopentylstruktur als Carbokationen bildende Komponente durchgeführt wird.

Als Edukte für das erfindungsgemäße Verfahren werden die entsprechenden Verbindungen der Formel I verwendet, bei denen ein Cyclohexanring oder mehrere Cyclohexanringe zumindest teilweise ciskonfiguriert vorliegen. In der Regel werden in das Verfahren cis/trans-Gemische eingesetzt, so wie sie aus den vorangehenden Synthesen entstehen. Der letzte Reaktionsschritt, der zur Ausgangsverbindung führt, ist meist eine Hydrierung, z.B. die Hydrierung von 4,4'-Dialkylbiphenyl, 1-Alkyl-4-(4-Alkyl-cyclohex-1-enyl)-benzol oder die Hydrierung von 1-Acyl-4-(4-alkylcyclohexyl)benzol zum cis/trans-Gemisch der entsprechenden 4,4'-Dialkylbicyclohexane:
Die Carbokationen bildende Verbindung (Katalysator) ist eine Verbindung, die unter den Reaktionsbedingungen geringe Mengen von carbokationischen Verbindungen erzeugt. Diese erfüllen die Rolle eines Katalysators. Die Carbokationen bildende Verbindung wird im Folgenden als Katalysator bezeichnet. Der Katalysator wird bevorzugt in substöchiometrischen Mengen eingesetzt, d.h. in der Praxis genügen wenige mol% (0,01 bis 15 mol%) bezogen auf die zu isomerisierende Verbindung, wobei diese Mengenangabe nicht einschränkend zu sehen ist. Abhängig von der Herstellung des Ausgangsmaterials ist in manchen Fällen ist eine Zugabe des Katalysators nicht nötig, weil bereits genügend Verunreinigungen enthalten sind, die die Rolle des Katalysators übernehmen. Dies kann insbesondere dann geschehen, wenn die Herstellung des Ausgangsmaterials über die Dehydratisierung eines Alkohols und anschließende Hydrierung oder über eine dehydratisierende Hydrierung verläuft und noch Spuren von Alkohol im zu isomerisierenden Gemisch verblieben sind. Bevorzugt wird der Katalysator jedoch eigens zugegeben.

Das Verfahren wird in einer bevorzugten Ausführungsform in Gegenwart eines sekundären oder tertiären Alkylhalogenids als Katalysator durchgeführt. Bevorzugt wird als Katalysator ein tertiäres Halogenid, ein Halogenid an einem Brückenkopf eines polycyclischen Sytems, wie z.B. Adamantan, oder ein Neopentylhalogenid (primäres Halogenid) eingesetzt. Als Halogenide werden in diesem Zusammenhang Chlor und Brom verwendet. Bei den Katalysatoren kann es sich auch um geminal mehrfach halogenierte Verbindungen, wie beispielsweise Haloform oder Tetrahalomethan, handeln.

Beispiele von guten Carbokationenbildnern sind brückenkopfsubstituierte Adamantanverbindungen wie z.B. 1-Halogenadamantan, außerdem Norbornylchlorid. Besonders wirtschaftlich aufgrund der guten Verfügbarkeit und Wirksamkeit ist die Verwendung von tert-Butylchlorid (2-Chlor-2-methylpropan) als Katalysator.

Eine weitere Alternative sind tert.-Carbonsäuren/säurechloride , wie z.B. Pivaloylchlorid (2,2-Dimethylpropanoylchlorid), weil es sich nach Abspaltung eines Chlorids unter Abspaltung von CO ein tertiäres Carbokation bilden kann.

Darüber hinaus zeigen auch Acyl- oder Aroylkationen (R-CO⁺ oder Ar-CO⁺) in Kombination mit Lewis-Säuren (beispielsweise AlCl₃ oder AlBr₃) die gewünschten katalytischen Eigenschaften. Katalytisch wirksame Systeme erhält man aus geeigneten Säurederivaten (Carbonsäuren und deren Säurechloriden bzw. Anhydriden) beispielsweise in situ durch Einwirkung von Lewis-Säuren, beispielweise Aluminiumhalogeniden.

Nachfolgend wird die beteiligte Lewis-Säure bzw. die ionische Flüssigkeit mit Lewis-saurem Anion auch als Aktivator bezeichnet. Der Aktivator wird bevorzugt in substöchiometrischen Mengen eingesetzt, d.h. in der Praxis werden bevorzugt Mengen zwischen 20 mol% und 0,05 mol% bezogen auf ein Mol des zu isomerisierenden Substrats eingesetzt. Besonders bevorzugt ist eine Menge an zugesetztem Aktivator von 1-6 mol%.

Der Katalysator wird bevorzugt ebenfalls in substöchiometrischen Mengen eingesetzt, insbesondere in einer Menge, die noch geringer ist als die des eingesetzten Aktivators. Bevorzugte Mengen des Katalysators sind 0,01 bis 5 mol% bezogen auf das Produkt oder 1 bis 90 mol%, bevorzugt 5 bis 20 mol% bezogen auf den Aktivator. Diese Mengenangaben sind nicht einschränkend zu sehen. Die Zugabe von zu großen oder gar stöchiometrischen Mengen an Katalysator und/oder Aktivator kann jedoch von Nachteil für das Verfahren sein, weil sich Nebenprodukte bilden.

Bei Acyl- oder Aroylhalogeniden als Katalysator ist bevorzugt AlCl₃ oder AlBr₃ der Aktivator. Das Molverhältnis von Aktivator zu Katalysator ist dann bevorzugt größer 1/1 , besonders bevorzugt 2/1 und größer.

Das Verfahren wird bevorzugt in einem chlorierten oder fluorierten Lösungsmittel, wie z.B. in Dichlormethan, mehrfach fluorierten Aromaten (z. B. 1,3-Difluorbenzol, 1,2,3,4- oder 1,2,3,5-Tetrafluorbenzol, Pentafluorbenzol) oder chlorierten Fluorkohlenwasserstoffen durchgeführt.

Die Reaktionstemperatur im Verfahren beträgt bevorzugt unter 20 °C, besonders bevorzugt unter 0 °C. Sie beträgt bevorzugt von 20 bis -180 °C, besonders bevorzugt von 0 °C bis -100 °C und ganz besonders bevorzugt von -30 °C bis -78 °C. Durch die niedrige Verfahrenstemperatur werden wenig Nebenprodukte erzeugt. Auch empfindliche Edukte können eingesetzt werden.

Das gewünschte Produkt wird in der Regel nach einer Reaktionszeit von 0,1 h bis 4 h erhalten.

Das erfindungsgemäße Verfahren zeichnet sich durch eine schonende Verfahrensweise aus, durch einen äußerst hohen trans-Anteil im Isomerisierungsprodukt und durch wenig Nebenprodukte. Die Abtrennung des zugesetzten Katalysators ist unproblematisch.

Das erfindungsgemäße Verfahren stellt eine effiziente Methode zur Isomerisierung des unerwünschten cis-Anteils der 1,4-Cyclohexanderivate dar. Das Verfahren kann zur direkten Erhöhung des trans-Anteils von cis/trans-Gemischen verwendet werden, selbst wenn der Anteil des trans-Isomeren bereits 85 % oder mehr beträgt. Der trans-Anteil nach der Isomerisierung beträgt vorzugsweise 94 % oder mehr, besonders bevorzugt 97 % oder mehr und insbesondere 99 % oder mehr. Der Gehalt bezieht sich auf das Rohgemisch vor der Kristallisation oder Aufreinigung. Ebenso kann das Verfahren zur Isomerisierung von Rückständen mit erhöhtem cis-Anteil aus der Anreicherung von trans-Isomeren, z.B. Mutterlaugen aus der Kristallisation, verwendet werden. So können auch nicht verwertbare Reste an cis-konfiguriertem Material durch Isomerisierung wiederverwertet werden, oder ihre Entstehung wird vermieden. Die gewonnenen trans-Cyclohexanverbindungen sind beispielsweise wertvolle Komponenten von LCD-Anzeigen.

Ein bevorzugtes Verfahren ist daher dadurch gekennzeichnet, dass die Konfiguration des Reaktionsprodukts an jedem 1,4-substituierten Cyclohexanring zu 94 % oder mehr der trans-Konfiguration entspricht, wobei die Konfiguration des Edukts an mindestens einem 1,4-substituierten Cyclohexanring zu 90 % oder weniger der cis-Konfiguration entspricht.

Als Aktivator sind solche Lewis-Säuren einsetzbar, die die klassischen Friedel-Crafts Alkylierungen katalysieren. An erster Stelle seien die Halogenide der dritten und fünften Hauptgruppe und der Lanthaniden zu nennen, von denen AlCl₃, AlBr₃ und BF₃ und SbCl₅ besonders bevorzugt sind. Bekannte Friedel-Crafts Katalysatoren sind auch Halogenide wie z.B. FeCl₃, SnCl₄ oder ZnBr₂. Wie die Verbindung B(OCOCF₃)₃ zeigt, sind neben den Halogeniden auch Sauerstoffkomplexe geeignete Aktivatoren.

Wegen der einfachen Handhabbarkeit sind flüssige Lewis-Säuren, wie man sie in ionischen Flüssigkeiten ('Ionic Liquids') findet, besonders bevorzugt. Solche 'Ionic Liquids' setzen sich zusammen aus einem organischen "Onium-Kation", wie z. B. Tetraalkyammonium, Tetraalkylphosphonium oder Trialkylsulfonium und einem Lewis-sauren Anion. Darüber hinaus bilden neben alkyliertem Guanidinium auch N-alkylierte Heterocyclen, wie z. B. N-Alkyl-Pyridinium, N,N'-Dialkylimidazolium (1,3-Dialkylimidazolium) oder N,N-Dialkylpyrrolidinium geeignete Kationen für 'Ionic Liquids'. Entscheidend für die Wirkung als Lewis-saurer Aktivator ist aber das Anion. Dieses setzt sich zusammen aus dem "at"-Komplex einer Lewis-Säure, wie z.B. AlCl₄⁻ und der Lewis-Säure selbst, also AlCl₃. Daraus ergibt sich dann mit 1/1 Stöchiometrie das Lewis-saure Anion Al₂Cl₇, wobei die 1/1-Stöchiometrie nicht Voraussetzung ist. Es kann durchaus auch weniger oder mehr AlCl₃ eingesetzt werden als der Menge an AlCl₄⁻ entspricht. Bevorzugt ist aber die stöchiometrische 1/1-Menge der Lewis-Säure oder etwas weniger. Andere analoge Anionen sind beispielsweise In₂Cl₇⁻, Fe₂Cl₇⁻ oder Sb₂F₁₁⁻.

In einer bevorzugten Ausführungsform der Erfindung wird das erfindungsgemäße Verfahren für Verbindungen der Formel I angewendet, worin m = 1 und n=0 ist. Für den Fall, dass die Formel I zwei Cyclohexanringe aufweist, eignet sich das erfindungsgemäße Verfahren besonders. Dabei kann auch bereits einer der Cyclohexanringe die gewünschte trans-Konfiguration aufweisen, weil diese erhalten bleibt. Genauso ist es möglich, durch die hohe trans-Selektivität des Isomerisierungsverfahrens auch mehrere Isomerisierungen an 1,4-substituierten Cyclohexanringen gleichzeitig durchzuführen. Der Anteil der resultierenden all-trans-Produkte ist in der Regel hoch genug, um eine anschließende Abtrennung von cis-Derivaten ohne großen Verlust in einem Kristallisationsschritt zu erreichen . Vergleichsweise werden ohne ein erfindungsgemäßes effektives Isomerisierungsverfahren zur Abtrennung der unerwünschten cis-Isomere in der Regel mehrere Kristallisationsschritte benötigt, und die Kristallisation des all-trans-Produkts wird durch das anwesende cis-Isomer und eventuell auftretende smektische Phasen erschwert.

Die im erfindungsgemäßen Verfahren eingesetzten Cyclohexane mit einem zu isomerisierenden cis-Anteil werden nach üblichen Methoden hergestellt. Bewährte Ausgangsverbindungen sind 4-substituierte Cyclohexanone, beispielsweise 4-(4-Alkylcyclohexyl)cyclohexanone. Aus diesen Edukten bieten sich zweierlei Synthesewege zu den Cyclohexanen an: Durch Addition von Grignard- oder Lithium-Verbindungen an die Carbonylgruppe mit anschließender Eliminierung und Hydrierung der so erhaltenen Alkene an gängigen Katalysatoren oder durch Wittig-Reaktion mit Alkylphosphoniumsalzen und anschließender Hydrierung. Aus den jeweiligen Hydrierungen resultieren in der Regel cis/trans-Gemische mit einem trans-Gehalt kleiner 85 %, meistens wesentlich darunter. Ein bevorzugtes Verfahren ist daher dadurch gekennzeichnet, dass nach der Isomerisierung die Konfiguration des Reaktionsprodukts an jedem 1,4-substituierten Cyclohexanring zu 94 % oder mehr der trans-Konfiguration entspricht. Dabei entspricht vorzugsweise die Konfiguration des Edukts an mindestens einem 1,4-substituierten Cyclohexanring zu 90 % oder weniger der trans-Konfiguration. Bevorzugt entspricht die Konfiguration des Reaktionsprodukts nach der Isomerisierung zu 94 % und mehr, besonders bevorzugt zu 97 % und mehr und ganz besonders bevorzugt 99% oder mehr der all-trans-Konfiguration.

Eine weitere Synthese der zu isomerisierenden Cyclohexanverbindungen stellt die Hydrierung von entsprechenden Benzolverbindungen dar, beispielsweise von substituierten Cyclohexylbenzolen. Diese Verbindungsklasse lässt sich leicht aus chemischen Grundverbindungen erhalten. Geeignete Hydrierkatalysatoren und die Verfahrensparameter sind dem Fachmann vertraut.

Ein bevorzugtes Verfahren gemäß der Erfindung ist daher dadurch gekennzeichnet, dass es als einen weiteren Verfahrensschritt vor der erfindungsgemäßen Isomerisierung eine Hydrierung an einem Benzolring, einem Cyclohexenring oder einem Cyclohexadienring umfasst, wobei dieser Ring in einen Cyclohexanring überführt wird. Vorzugsweise wird genau dieser Ring bei der Isomerisierung in die trans-Konfiguration überführt, soweit er in der cis-Konfiguration oder als cis/trans Gemisch aus der Hydrierung hervorgeht. Vorzugsweise erfolgt die Isomerisierung direkt nach der Hydrierung.

Für den Fall, dass die Formel I mehr als zwei Cyclohexanringe aufweist eignet sich das erfindungsgemäße Verfahren ebenfalls. Dabei kann auch bereits einer oder zwei der Cyclohexanringe die gewünschte trans-Konfiguration aufweisen, weil diese erhalten bleibt. Genauso ist es möglich, durch die hohe trans-Selektivität des Isomerisierungsverfahrens auch Isomerisierungen an drei Ringen gleichzeitig durchzuführen.

Der Ausdruck "Alkyl" umfasst vorzugsweise unverzweigte oder verzweigte Alkylgruppen mit 1 bis 15 Kohlenstoffatomen, insbesondere die unverzweigten Gruppen Methyl, Ethyl, Propyl, Butyl, Pentyl, Hexyl und Heptyl. Gruppen mit 2 bis 10 Kohlenstoffatomen sind im allgemeinen bevorzugt. Als verzweigte Alkylgruppe an Verbindungen der Formel I ist die Isopropylgruppe bevorzugt.

Der Ausdruck "Aroyl" umfasst Verbindungen der Formel "Aryl"-(CO)-. "Aryl" umfasst aromatische Reste, die auch durch Alkyl substituiert sein können, insbesondere Phenyl oder p-Tolyl.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z.B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

In den Diagrammen bedeuten die Ringe

| | |
|---|---|
| 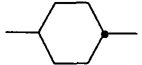 | (mit Punkt) einen 1,4-trans-substituierten Cyclohexanring, und |
| 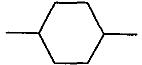 | einen 1,4-substituierten Cyclohexanring mit gemischter cis- und trans-Konfiguration oder überwiegend cis-Konfiguration. |

### Beispiele

### Allgemeines

Die Auswertung der entnommen Proben und die Bestimmung der Konfiguration des Endprodukts erfolgt mittels HPLC mit Acetonitril als Lösungsmittel an einer Purospher® STAR RP-18 Säule (Merck KGaA, Darmstadt). Die Zwischenproben werden mit Salzsäure hydrolysiert, mit Bicarbonat neutralisiert, extrahiert eingedampft und vermessen.

### Beispiel 1

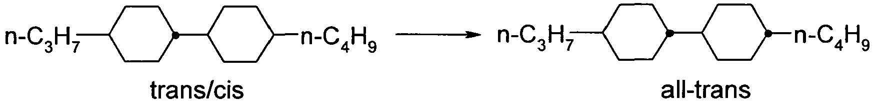

In 50 ml wasserfreiem Dichlormethan (zur Analyse) wird 1,67g (12 mmol) Aluminiumchlorid (wasserfrei, sublimiert und gepulvert) gegeben und auf -55 °C abgekühlt. Zwischen -49 und -55 °C wird 4-Propyl-4'-butyl-bicyclohexan (26,4 g, 100 mmol, 16,4% cis-Anteil) während 20 min zugetropft. Anschließend werden 0,2 ml (1,8 mmol) 2-Chlor-2-methylpropan zugegeben. Der Ansatz wird bei -55 °C weitere 2,5 h gerührt, wobei nach jeweils 15, 30, 60, 90 und 120 min eine Probe zur HPLC-Bestimmung des Isomerisierungsgrades entnommen wird.

Zur Aufarbeitung wird der Ansatz in eine Mischung aus 100 ml Salzsäure (25 %) und 50 g Eis eingerührt. Die organische Phase wird abgetrennt, mit 100 ml Wasser und dann mit Bicarbonatlösung gewaschen und am Rotationsverdampfer zum Rückstand eingeengt. Der Rückstand (22 g) enthält 1,8 % Anteil an Produkt mit einem cis-konfiguriertem Ring und 96,6 % an all-trans Produkt.

**Tabelle: Zeitlicher Verlauf des Isomerisierungsverfahrens**

| | | | | | | |
|---|---|---|---|---|---|---|
| Reaktionszeit [min] | 15 | 30 | 60 | 90 | 120 | 150(*) |
| all-trans-Anteil [%] | 98,9 | 98,1 | 97,3 | 97,3 | 96,9 | 96,6 |

| | | | | | | |
|---|---|---|---|---|---|---|
| (*) Isoliertes Rohprodukt | | | | | | |

### Beispiel 2

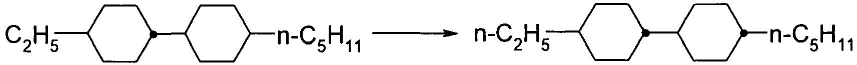

In 50 ml wasserfreiem Dichlormethan (zur Analyse) wird 2,74 g (6,2 mmol) N-Butylpyridiniumheptachloroaluminat gegeben und auf -55 °C abgekühlt. Zwischen -49 und -55 °C wird 4-Ethyl-4'-pentyl-bicyclohexan (26,4 g, 100 mmol, 36 % cis-Anteil) in 50 ml Dichlormethan während 20 min zugetropft. Anschließend werden 0,20 ml (1,8 mmol) 2-Chlor-2-methylpropan zugegeben. Der Ansatz wird bei -55 °C weitere 2,5 h gerührt, wobei nach jeweils 15 min eine Probe zur HPLC-Bestimmung des Isomerisierungsgrades entnommen wird.

Zur Aufarbeitung wird der Ansatz in eine Mischung aus 100 ml Salzsäure (25 %) und 50 g Eis eingerührt. Die organische Phase wird abgetrennt, mit 100 ml Wasser und dann mit Bicarbonatlösung gewaschen und am Rotationsverdampfer zum Rückstand (23,7 g) eingeengt.

**Tabelle: Zeitlicher Verlauf des Isomerisierungsverfahrens**

| | | | | | | |
|---|---|---|---|---|---|---|
| Reaktionszeit [min] | 15 | 30 | 60 | 90 | 120 | 150(*) |
| all-trans-Anteil [%] | 99,3 | 98,0 | 98,6 | 98,0 | 97,5 | 98,2 |

| | | | | | | |
|---|---|---|---|---|---|---|
| (*) Isoliertes Rohprodukt | | | | | | |

### Beispiel 3

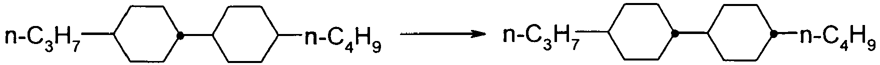

264,5 g (1 mol) Isomerengemisch (79,0 % trans, 21,0 % cis) 4-Propyl-4'-butyl-bicyclohexan und 2 ml (18 mmol) 2-Chlor-2-methylpropan werden in 1000 ml Dichlormethan bei -55°C vorgelegt. Dabei bildet sich eine Suspension. Unter fortgesetztem Kühlen gibt man dazu 2,5 ml (7,1 mmol) N-Butylpyridiniumheptachloroaluminat und rührt bei -55°C (+/-2°C) 150 Minuten. Dann hydrolysiert man mit einem Gemisch aus Eis (500 g) und 1000 ml 25 %iger Salzsäure. Die organische Phase wird abgetrennt und mit Wasser und anschließend mit NaHCO₃ Lösung und nochmals mit Wasser neutral gewaschen. Dann wird zum Rückstand (261,8 g) eingeengt (99,2 % all-trans Produkt).

**Tabelle: Zeitlicher Verlauf des Isomerisierungsverfahrens**

| | | | | | | |
|---|---|---|---|---|---|---|
| Reaktionszeit [min] | 15 | 30 | 60 | 90 | 120 | 150(*) |
| all-trans-Anteil [%] | 97,8 | 98,0 | 98,9 | 99,0 | 99,2 | 99,2 |

| | | | | | | |
|---|---|---|---|---|---|---|
| (*) isoliertes Rohprodukt | | | | | | |

### Beispiel 4

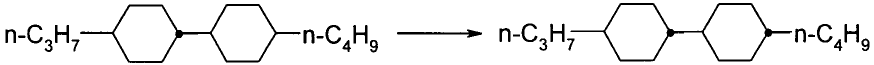

Durchführung wie Beispiel 3, jedoch mit Neopentylchlorid (4 mmol)

**Tabelle: Zeitlicher Verlauf des Isomerisierungsverfahrens**

| | | | | | | |
|---|---|---|---|---|---|---|
| Reaktionszeit [min] | 15 | 30 | 60 | 90 | 120 | 150(*) |
| all-trans-Anteil [%] | 72,2 | 93,3 | 99,3 | 99,5 | 99,1 | 99,5 |

| | | | | | | |
|---|---|---|---|---|---|---|
| (*) isoliertes Rohprodukt | | | | | | |

### Beispiel 5

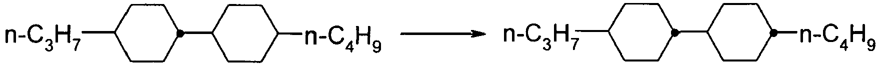

Durchführung wie Beispiel 2, jedoch mit 200 µl 1,3-Di-tert-butylbenzol (0,9 mmol).

**Tabelle: Zeitlicher Verlauf des Isomerisierungsverfahrens**

| | | | | |
|---|---|---|---|---|
| Reaktionszeit [min] | 30 | 60 | 120 | 120(*) |
| all-trans-Anteil [%] | 79,1 | 80,9 | 90,0 | 89,7 |

| | | | | |
|---|---|---|---|---|
| (*) isoliertes Rohprodukt | | | | |

### Beispiel 6

Es werden bei -40°C 1,33 g Aluminiumchlorid (0,01 mol) in 70 ml Dichlormethan vorgelegt. Dazu gibt man 23,6 g (0,1 mol) 4-Ethyl-4'-propylbicyclohexan als Isomerengemisch (41,5 % cis; 57,2 % trans) gelöst in 20 ml Dichlormethan. Es wird mit 10 ml Dichlormethan nachgespült. Bei -40°C gibt man dazu 2 mmol (157 mg, 143 µl) Acetylchlorid. Die Suspension wird gerührt und die Isomerisierung über HPLC verfolgt (Tabelle). Nach 240 min wird aufgearbeitet. Das gereingte Produkt hat einen all-trans-Anteil von 97,3 %.

**Tabelle: Zeitlicher Verlauf des Isomerisierungsverfahrens**

| | | | | | |
|---|---|---|---|---|---|
| Reaktionszeit [min] | 30 | 60 | 120 | 180 | 240 |
| all-trans-Anteil [%] | 69,6 | 97,7 | 98,7 | 97,1 | 97,1 |

### Beispiel 7

22,5 g 4-Ethyl-4'-propylbicyclohexan (cis/trans-Gemisch: trans 60,3 %/ cis 39,7 %) werden bei Raumtemperatur in 90 mL Dichlormethan vorgelegt, dann werden zu der Lösung 3,05 g Antimonpentachlorid gegeben. Unter Rühren wird auf -42°C abgekühlt, wobei eine rührbare Suspension entsteht. Zu dieser gibt man 180 mg 1-Chloradamantan gelöst in 10 mL Dichlormethan. Bereits nach 30 Minuten ist der HPLC-Gehalt einer Probe 95,8 % trans und bleibt über 4 Stunden konstant.

**Tabelle: Zeitlicher Verlauf des Isomerisierungsverfahrens**

| | |
|---|---|
| Reaktionszeit [min] | 30 |
| all-trans-Anteil [%] | 95,8 |

### Beispiel 8

23,7 g 4-Ethyl-4'-propylbicyclohexan (cis/trans-Gemisch: trans 60,3 %/ cis 39,7 %) werden bei Raumtemperatur in 90 mL Dichlormethan vorgelegt, dann werden zu der Lösung 1,65 g Eisen(III)-chlorid gegeben. Unter Rühren wird auf -42°C abgekühlt, wobei eine rührbare Suspension entsteht. Zu dieser gibt man 340 mg 1-Chloradamantan gelöst in 10 mL Dichlormethan.

**Tabelle: Zeitlicher Verlauf des Isomerisierungsverfahrens**

| | | |
|---|---|---|
| Reaktionszeit [min] | 120 | 240 |
| all-trans-Anteil [%] | 73,5 | 82,6 |

## Patentansprüche

1. Verfahren zur Herstellung von 1,4-trans-substituierten Cyclohexanverbindungen der Formel I
R¹-[A¹-(CH₂)ₙ-]ₘ-A²-R² I
worin
R¹ und R² unabhängig voneinander einen unsubstituierten, geradkettigen Alkylrest mit bis zu 9 C-Atomen oder -CH(CH₃)CH₃,
m 1 oder 2,
n jeweils unabhägig voneinander 0, 1, 2, 3 oder 4, und
A¹, A² *trans*-1,4-Cyclohexylen,
bedeuten,
das die Umsetzung von Verbindungen der Formel I entsprechenden 1,4-cis-Cyclohexanverbindungen in Gegenwart
a) einer Lewis-Säure oder
b) eines Lewis-sauren Anions,
und in Gegenwart einer zusätzlichen Komponente, die zusammen mit der Lewis-Säure bzw. mit dem Lewis-sauren Anion ein Carbokation bilden kann, umfasst,
**dadurch gekennzeichnet, dass** es in Gegenwart eines sekundären oder tertiären Alkylchlorids oder Alkylbromids, eines Acyl- oder Aroylhalogenids oder eines primären Halogenids mit Neopentylstruktur als Carbokationen bildende Komponente durchgeführt wird.

2. Verfahren nach Anspruch 1 , **dadurch gekennzeichnet, dass** als Lewis-Säure AlCl₃ oder AlBr₃ verwendet wird.

3. Verfahren nach nach Anspruch 1 oder 2, **dadurch gekennzeichnet, dass** die Reaktion in Gegenwart eines tertiären oder sekundären Alkylhalogenids oder in Gegenwart eines primären Halogenids mit Neopentylstrukturdurchgeführt wird.

4. Verfahren nach einem oder mehreren der Ansprüche 1 bis 3, **dadurch gekennzeichnet, dass** trans/trans-Bicyclohexan-Verbindungen der Formel IA 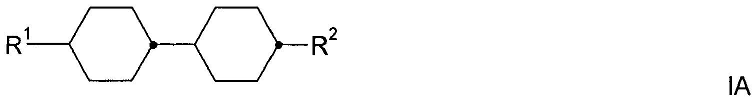 worin R¹ und R² wie in Anspruch 1 definiert sind,
hergestellt werden.

5. Verfahren einem oder mehreren der Ansprüche 1 bis 4, **dadurch gekennzeichnet, dass** die Carbokationen bildende Verbindung ein brückenkopfsubstituiertes Adamantan ist.

6. Verfahren nach einem oder mehreren der Ansprüche 1 bis 5, **dadurch gekennzeichnet, dass** die Reaktion bei Temperaturen kleiner oder gleich 20 °C durchgeführt wird.

7. Verfahren nach einem oder mehreren der Ansprüche 1 bis 6, **dadurch gekennzeichnet, dass** die Reaktion in einem chlorierten und/oder fluorierten Kohlenwasserstoff als Lösungsmittel durchgeführt wird.

8. Verfahren nach einem oder mehreren der Ansprüche 1 bis 9, **dadurch gekennzeichnet, dass** es als einen Verfahrensschritt vor der Isomerisierung hinführend zu den Verbindungen der Formel I eine Hydrierung umfasst, die an einem Benzolring, einem Cyclohexenring oder einem Cyclohexadienring erfolgt, wobei diese Ringe in einen 1,4-substituierten Cyclohexanring überführt werden, oder eine Hydrierung, die an einer direkt an den Cyclohexanring gebundenen Alkylidengruppe erfolgt.

## Claims

1. Process for the preparation of 1,4-trans-substituted cyclohexane compounds of the formula I
R¹-[A¹-(CH₂)ₙ-]ₘ-A²-R² I
in which
R¹ and R², independently of one another, denote an unsubstituted, straight-chain alkyl radical having up to 9 C atoms or -CH(CH₃)CH₃,
m denotes 1 or 2,
n in each case, independently of one another, denotes 0, 1, 2, 3 or 4, and
A¹, A² denote *trans*-1,4-cyclohexylene,
which comprises the reaction of 1,4-cis-cyclohexane compounds corresponding to compounds of the formula I in the presence of
a) a Lewis acid or
b) a Lewis-acidic anion,
and in the presence of an additional component which is able to form a carbocation together with the Lewis acid or the Lewis-acidic anion, **characterised in that** it is carried out in the presence of a secondary or tertiary alkyl chloride or alkyl bromide, an acyl or aroyl halide or a primary halide having a neopentyl structure as carbocation-forming component.

2. Process according to Claim 1, **characterised in that** the Lewis acid used is AlCl₃ or AlBr₃.

3. Process according to Claim 1 or 2, **characterised in that** the reaction is carried out in the presence of a tertiary or secondary alkyl halide or in the presence of a primary halide having a neopentyl structure.

4. Process according to one or more of Claims 1 to 3, **characterised in that** trans/trans-bicyclohexane compounds of the formula IA 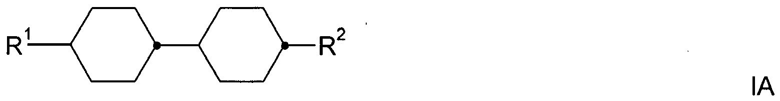 in which R¹ and R² are as defined in Claim 1,
are prepared.

5. Process according to one or more of Claims 1 to 4, **characterised in that** the carbocation-forming compound is a bridgehead-substituted adamantane.

6. Process according to one or more of Claims 1 to 5, **characterised in that** the reaction is carried out at temperatures of less than or equal to 20°C.

7. Process according to one or more of Claims 1 to 6, **characterised in that** the reaction is carried out in a chlorinated and/or fluorinated hydrocarbon as solvent.

8. Process according to one or more of Claims 1 to 7, **characterised in that** it comprises, as a process step before the isomerisation leading to the compounds of the formula I, a hydrogenation which is carried out on a benzene ring, a cyclohexene ring or a cyclohexadiene ring, where these rings are converted into a 1,4-substituted cyclohexane ring, or a hydrogenation which is carried out an alkylidene group bonded directly to the cyclohexane ring.

## Revendications

1. Procédé de préparation de composés de cyclohexane 1,4-trans-substitué de formule I
R¹-[A¹-(CH₂)ₙ-]ₘ-A²-R² I
dans laquelle
R¹ et R², indépendamment l'un de l'autre, désignent un radical alkyle à chaîne linéaire non substitué, ayant jusqu'à 9 atomes de C ou -CH(CH₃)CH₃,
m désigne 1 ou 2,
n dans chaque cas, indépendamment l'un de l'autre, désigne 0, 1, 2, 3 ou 4, et
A¹, A² désignent *trans*-1,4-cyclohexylène,
comprenant la réaction de composés de 1,4-cis-cyclohexane correspondant aux composés de formule I en présence
a) d'un acide de Lewis ou
b) d'un anion d'acide de Lewis,
et en présence d'un composant supplémentaire qui est capable de former un carbocation conjointement avec l'acide de Lewis ou l'anion d'acide de Lewis,
**caractérisé en ce qu'**elle est effectuée en présence d'un chlorure d'alkyle ou bromure d'alkyle secondaire ou tertiaire, un halogénure d'acyle ou d'aroyle ou un halogénure primaire ayant une structure de néopentyle comme composant formateur de carbocation.

2. Procédé selon la revendication 1, **caractérisé en ce que** l'acide de Lewis utilisé est AlCl₃ ou AlBr₃.

3. Procédé selon la revendication 1 ou 2, **caractérisé en ce que** la réaction est effectuée en présence d'un halogénure d'alkyle tertiaire ou secondaire ou en présence d'un halogénure primaire ayant une structure de néopentyle.

4. Procédé selon l'une ou plusieurs parmi les revendications 1 à 3, **caractérisé en ce que** les composés de trans/trans-bicyclohexane de formule IA 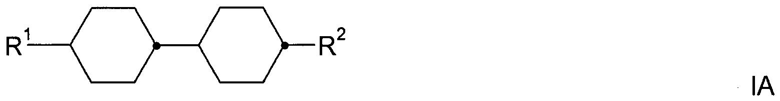 dans laquelle R¹ et R² sont tels que définis selon la revendication 1,
sont préparés.

5. Procédé selon l'une ou plusieurs parmi les revendications 1 à 4, **caractérisé en ce que** le composé formateur de carbocation est un adamantane substitué en tête de pont.

6. Procédé selon l'une ou plusieurs parmi les revendications 1 à 5, **caractérisé en ce que** la réaction est effectuée à des températures inférieures ou égales à 20°C.

7. Procédé selon l'une ou plusieurs parmi les revendications 1 à 6, **caractérisé en ce que** la réaction est effectuée dans un hydrocarbure chloré et/ou fluoré comme solvant.

8. Procédé selon l'une ou plusieurs parmi les revendications 1 à 7, **caractérisé en ce qu'**il comprend, comme étape de procédé avant l'isomérisation conduisant aux composés de formule 1, une hydrogénation qui est effectuée sur un cycle benzène, un cycle cyclohexène ou un cycle cyclohexadiène, où ces cycles sont convertis en cycle cyclohexane 1,4-substitué, ou une hydrogénation qui est effectuée sur un groupement alkylidène lié directement au cycle cyclohexane.
